# EUROPÄISCHE PATENTSCHRIFT

(11) **EP 4 389 023 B1**
(45) Veröffentlichungstag und Bekanntmachung des Hinweises auf die Patenterteilung: **15.10.2025**
(21) Anmeldenummer: 23216967.2
(22) Anmeldetag: 15.12.2023
(51) Int. Cl.: A61B 17/29, A61B 34/00, A61B 17/00

(54) **LENKGETRIEBE FÜR EIN CHIRURGISCHES INSTRUMENT UND DAMIT AUSGESTATTETES CHIRURGISCHES INSTRUMENT**
STEERING GEAR FOR SURGICAL INSTRUMENT AND SURGICAL INSTRUMENT EQUIPPED WITH THE SAME
MÉCANISME DE DIRECTION POUR INSTRUMENT CHIRURGICAL ET INSTRUMENT CHIRURGICAL ÉQUIPÉ DE CELUI-CI

(30) Priorität: 20.12.2022 DE 102022134210
(43) Veröffentlichungstag der Anmeldung: 26.06.2024
(73) Patentinhaber: Karl Storz SE & Co. KG, 78532 Tuttlingen (DE)
(72) Erfinder: Grüner, Sven, 78532 Tuttlingen (DE)

(56) Entgegenhaltungen:
- WO-A1-2023/006684
- WO-A1-2023/006685
- DE-A1- 102019 121 092
- US-B2- 10 105 128

## Beschreibung

Die Erfindung betrifft ein Lenkgetriebe eines chirurgischen Instruments sowie ein chirurgisches Instrument, das ein solches Lenkgetriebe aufweist.

Aus dem Stand der Technik sind chirurgische Instrumente bekannt, die mittels einer Handhabe manuell oder von einem Roboter geführt werden können und am distalen Ende eines länglichen Schafts ein Werkzeug aufweisen, das durch eine Abwinkelungsmechanik aus mehreren ineinandergreifenden Schwenkgliedern gegenüber dem Schaft, der eine Hauptachse definiert, verschwenkt werden kann. Diese Schwenkglieder sind mit einer Vielzahl Lenkdrähte oder - seile verbunden, um eine feinfühlige Steuerung der Werkzeugspitze zu erreichen. Zur Betätigung können die Lenkdrähte an einer kardanisch gelagerten Taumelscheibe befestigt sein, die sich durch ein Lenkgetriebe räumlich ausrichten lässt.

Aus US 10,105,128 B2 ist bekannt, eine über Lenkdrähte mit einer Abwinkelungsmechanik verbundene kardanisch gelagerte Taumelscheibe mit zwei parallelen Stangen zu betätigen. Dazu weisen die Stangen an einem Ende eine Kugelpfanne zur Aufnahme eines mit der Taumelscheibe gekoppelten Kugelelements auf. An ihrem anderen Ende sind diese Kugelgelenkstangen über Drehgelenke mit jeweils einem durch ein Antriebszahnrad betätigten Zahnradquadranten verbunden, um die Kugelgelenkstangen zur Ausrichtung der Taumelscheibe linear vor und zurück zu bewegen. Allerdings ist die Taumelscheibenansteuerung mit Kugelgelenkstangen nicht sonderlich direkt, sondern spielbehaftet und weist einen ungünstigen Kraftfluss auf. Zudem ist eine Rotation der Taumelscheibe um die Schaftachse hierbei nicht möglich.

Dazu schlägt DE 10 2019 121 092 A1 ein chirurgisches Instrument vor, das ein Differenzialgetriebe mit zwei gegenüberliegenden Antriebskegelrädern und einem Abtriebskegelrad einsetzt, das mit den Antriebskegelrädern in Eingriff steht und mit der Taumelscheibe gekoppelt ist. Auf diese Weise werden die Stellwinkel von zwei Antrieben direkt auf die Taumelscheibe übertragen, um die Werkzeugspitze entsprechend auszurichten bzw. abzuwinkeln. Ferner ist dort die Taumelscheibe mittels einer Kreuzgelenkscheibe kardanisch auf einer drehbaren Hauptwelle gelagert und kann daher mit der Hauptwelle um die Schaftachse rotiert werden. Allerdings hat die koaxiale, in einer Ebene gegenüberliegende Anordnung der beiden Antriebe orthogonal zu der Hauptachse einen erhöhten Bauraumbedarf.

Zum weiteren Stand der Technik kann auf die nachveröffentlichten Dokumente WO 2023/006684 A1 und WO 2023/006685 A1 verwiesen werden, welche unter Art. 54 (3) EPÜ fallen.

Ausgehend von diesem Stand der Technik ist es Aufgabe der vorliegenden Erfindung, ein verbessertes Lenkgetriebe für ein chirurgisches Instrument zur räumlichen Ausrichtung einer Taumelscheibe bereitzustellen.

Diese Aufgabe wird durch ein Lenkgetriebe mit den Merkmalen des Anspruchs 1 gelöst.

Die weitere Aufgabe, ein chirurgisches Instrument mit einem verbesserten Lenkgetriebe bereitzustellen, wird durch das chirurgische Instrument mit den Merkmalen des unabhängigen Anspruchs 11 gelöst.

Weiterbildungen bzw. bevorzugte Ausführungsformen sind in den Unteransprüchen ausgeführt.

Gemäß einer ersten Ausführungsform weist das erfindungsgemäße Lenkgetriebe, das für ein chirurgisches Instrument vorgesehen und ausgebildet ist, zumindest zwei Antriebe zur räumlichen Ausrichtung einer Taumelscheibe auf. Die Taumelscheibe ist mit einer Hauptwelle rotativ gekoppelt, die eine Hauptachse A definiert, um die die Hauptwelle rotierbar ist. Aufgrund der Kopplung kann die Taumelscheibe gemeinsam mit der Hauptwelle rotieren. Ferner ist die Taumelscheibe kardanisch um ein Zentrum gelagert, das auf der Hauptachse A liegt. Erfindungsgemäß weist das Lenkgetriebe zumindest zwei Linearschieber auf, die jeweils entlang einer zu der Hauptachse A parallelen Führungsachse Bₓ verschiebbar angeordnet und mit den zumindest zwei Antrieben verbunden sind. Jeder Linearschieber steht mit der Taumelscheibe über ein Eingriffselementepaar in Wirkverbindung. Die Eingriffselemente des Paares werden durch eine Eingriffsöffnung und ein Hebelelement gebildet. Das Hebelelement hat an einem freien Ende eines Stababschnitts einen Kopfabschnitt zur beweglichen Aufnahme in der Eingriffsöffnung. Dabei liegt eines der Eingriffselemente des Eingriffselementepaars an einem Umfang der Taumelscheibe und das andere Eingriffselement des Eingriffselementepaars an einem der Taumelscheibe zugewandten Oberflächenabschnitt des jeweiligen Linearschiebers vor, der ein Oberflächenabschnitt an einer Längsseite des Linearschiebers parallel zur Führungsachse Bₓ ist. Auf diese Weise befindet sich der Kopfabschnitt jedes Hebelelements auf einer Umfangslinie um das Taumelscheibenzentrum, da die Wirkverbindung der Taumelscheibe mit den zumindest zwei Linearschiebern folglich zumindest zwei Hebelelemente aufweist, sodass eine Schwenkebene der Taumelscheibe durch die Kopfabschnitte der Hebelelemente und das Zentrum der Taumelscheibe definiert wird.

Mit dem erfindungsgemäßen Lenkgetriebe ist es möglich, die Taumelscheibe zur Ansteuerung einer distalen Abwinkelungsmechanik direkt durch Linearantriebe in zwei Raumrichtungen zu verschwenken, wobei die Taumelscheibe zusätzlich gemeinsam mit der Hauptwelle rotieren kann. Dies sorgt dafür, dass die an der Taumelscheibe befestigten Lenkdrähte bei Drehung der Hauptwelle mitgedreht werden und sich nicht um die Hauptwelle wickeln können. Die Taumelscheibe kann dabei sowohl in neutraler Stellung, d. h. im unartikulierten Zustand, wenn die Schwenkebene der Taumelscheibe orthogonal zu der Hauptachse A steht, mit der Hauptwelle rotieren, als auch nach erfolgter Artikulation in verschwenkter Stellung, in der die Hauptachse A die Schwenkebene der Taumelscheibe unter einem von 90° abweichenden Winkel schneidet. Um die Taumelscheibe aus der neutralen Stellung zu verschwenken, wird zumindest einer der Linearschieber entlang der Führungsachse Bₓ parallel zur Hauptachse A bewegt, wobei das Hebelelement als stabförmiges Kraftübertragungselement des jeweiligen Eingriffelementepaars der Bewegung des Linearschiebers folgt. Mit dieser Bewegung ändern sich die Lage des Kopfabschnitts des Hebelelements und damit die Lage der Schwenkebene, sodass die Taumelscheibe gegenüber der Hauptachse A um ihr Zentrum entsprechend ausgelenkt wird. Das Zentrum der Taumelscheibe, um das die Taumelscheibe ferner durch die Kopplung mit der Hauptwelle rotieren kann, wird durch den Schnittpunkt der Drehachsen definiert, die sich aus der kardanischen Lagerung ergeben.

Als "kardanische Lagerung" wird vorliegend im Sinne der Erfindung jegliche Lagerung der Taumelscheibe verstanden, die ein Verschwenken der rotativ mit der Hauptwelle gekoppelten Taumelscheibe in zwei Richtungen orthogonal zur Hauptachse (Kippen und Gieren) gestattet. Dies umfasst sowohl herkömmliche Lagervarianten mit zwei orthogonalen Achspaaren als auch Lagervarianten mit abweichender Anzahl und Anordnung von Achselementen, sofern diese eben Bewegungen der Taumelscheibe in zwei Raumrichtungen orthogonal zur Hauptachse A gestatten.

Der Index x bei der Bezeichnung der Führungsachse Bₓ steht stellvertretend für eine natürliche Zahl (1, 2, 3 oder 4) und wird vorliegend zur vereinfachten Bezeichnung der jeweils einem Linearschieber zugeordneten Führungsachsen Bₓ verwendet. Mit Index 1 ist die Führungsachse B₁ einem ersten Linearschieber zugeordnet. Eine Führungsachse B₂ ist einem zweiten Linearschieber zugeordnet, usw.

Nach einer weiteren Ausführungsform des erfindungsgemäßen Lenkgetriebes ist der Kopfabschnitt ein Kugelkopfabschnitt, der zumindest teilweise eine Kugelform aufweist, oder ein T-Kopfabschnitt, der zumindest teilweise zylinderartig geformt ist. In der Ausführungsform des Hebelelements mit dem T-Kopfabschnitt kann vorgesehen sein, dass das Hebelelement drehbar um die Längsachse des Stababschnitts oder der T-Kopfabschnitt drehbar an dem Stababschnitt gelagert ist, um der Bewegung der Linearschieber im Eingriff mit der Taumelscheibe folgen zu können. Unter einem "T-Kopfabschnitt" wird verstanden, dass der zylinderähnlich geformte T-Kopfabschnitt orthogonal zu dem Stababschnitt verläuft und mittig auf dem Stababschnitt angeordnet ist, sodass der Stababschnitt und der Kopfabschnitt in der Seitenansicht eine T-Form aufweisen.

Gemäß einer weiteren Ausführungsform des erfindungsgemäßen Lenkgetriebes ist vorgesehen, dass die Taumelscheibe die Eingriffsöffnung und jeder Linearschieber jeweils eines der Hebelelemente aufweist. Dazu ist die Eingriffsöffnung entweder als eine entlang des Umfangrands der Taumelscheibe umlaufende Umfangsnut oder als ein entlang des Umfangrands der Taumelscheibe umlaufender Umfangsabsatz ausgebildet. Das Hebelelement ist dann an dem der Taumelscheibe zugewandten Oberflächenabschnitt des jeweiligen Linearschiebers angeordnet, sodass der Stababschnitt in radialer Richtung zur Hauptachse A weist und der Kopfabschnitt jedes Hebelelements in der Umfangsnut oder in dem Umfangsabsatz beweglich aufgenommen ist.

Mit der Umfangsnut bzw. dem Umfangsabsatz als gemeinsame Eingriffsöffnung für jedes Hebelelement kann auf aufwändige Kugelgelenkstangen verzichtet werden, um seitliche Verschiebungen auszugleichen. Im Unterschied zur Umfangsnut, die beidseitig begrenzt ist, ist der Umfangsabsatz nur einseitig begrenzt, wobei die Breite der Nut bzw. des Absatzes entsprechend zur geführten Aufnahme/Anlage des Kopfabschnitts der Hebelelemente dimensioniert ist. Die Mittelpunkte der Kopfabschnitte der Hebelelemente, deren Stababschnitte bzw. Längsachsen in radialer Richtung zur Hauptachse A weisen, liegen durch die Führung in der Umfangsnut bzw. an dem Umfangsabsatz stets exakt auf der Schwenkebene der Taumelscheibe. Werden die Linearschieber bewegt, verschieben sich diese Mittelpunkte der Kopfabschnitte mit den Hebelelementen und führen so zu einer Verschwenkung der Taumelscheibe. Die Kopplung der Taumelscheibe mit der Hauptwelle zur gemeinsamen Rotation um die Hauptwelle ist problemlos möglich, da die Umfangsnut bzw. der Umfangsabsatz umlaufend ist.

Um dabei bei Einsatz von Hebelelementen mit Kugelkopfabschnitt eine flächige Kraftübertragung zu ermöglichen, kann nach einer Weiterbildung des erfindungsgemäßen Lenkgetriebes das Eingriffselementepaar für jedes Hebelelement, das mit einem Kugelkopfabschnitt ausgebildet ist, ein Nutensteinelement aufweisen. Dieses Nutensteinelement ist zur Aufnahme in der umlaufenden Umfangsnut bzw. dem umlaufenden Umfangsabsatz ausgebildet und weist eine Kugelpfanne auf, in der der Kugelkopfabschnitt des Hebelelements beweglich aufgenommen werden kann. Optional können Nutensteinelemente, die den Hebelelementen benachbarter Linearschieber zugeordnet sind und daher in der Umfangsnut bzw. an dem Umfangsabsatz benachbart angeordnet sind, über jeweils ein elastisches Verbindungselement miteinander verbunden sein und zusammen ein Klammerelement bilden.

Ein solches Nutensteinelement kann analog zum Verlauf der Umfangsnut/des Umfangsabsatzes kreisbogenförmig gestaltet sein, wobei die Kugelpfanne zur gelenkigen Aufnahme des Kugelkopfabschnitts vorzugsweise mittig in dem Nutensteinelement platziert ist. Hebelelemente, die einen T-Kopfabschnitt aufweisen, können auf ein solches Nutensteinelement verzichten, da die T-Kopfabschnitte im Unterschied zu einem Kugelkopfabschnitt, dessen Kraftübertragung punktförmig ist, Kraft durch Linienberührung übertragen.

Gemäß einer weiteren Ausführungsform des erfindungsgemäßen Lenkgetriebes, in der die Taumelscheibe mit der umlaufenden Umfangsnut als Eingriffsöffnung ausgebildet ist, kann das Lenkgetriebe zwei Linearschieber aufweisen, an denen jeweils ein Hebelelement angeordnet ist. Dabei sind die Anordnung der Hebelelemente an den Linearschiebern und die Anordnung der zwei Linearschieber in Bezug zu der Taumelscheibe so aufeinander abgestimmt, dass zwei unterschiedlich orientierte Schwenkachsen C₁, C₂durch jeden der zwei Kopfabschnitte der beiden Hebelelemente mit dem Zentrum der Taumelscheibe definiert werden. D. h. dass zwischen beiden Schwenkachsen C₁, C₂ ein Winkel aufgespannt wird, der weder 0° noch 180° beträgt. In einer bevorzugten Ausführungsform verlaufen die beiden Schwenkachsen C₁, C₂ orthogonal zueinander, wenn sich die Taumelscheibe in Neutralstellung befindet, in der die Taumelscheibe im unausgelenkten Zustand orthogonal zur Hauptachse A steht.

Gemäß einer dazu alternativen Ausführungsform des erfindungsgemäßen Lenkgetriebes, in der die Taumelscheibe mit dem umlaufenden Umfangsabsatz als Eingriffsöffnung ausgebildet ist, weist das Lenkgetriebe drei Linearschieber mit j eweils einem Hebelelement und drei Antriebe auf, die mit den Linearschiebern verbunden sind. Dabei sind die Anordnung der Hebelelemente an den Linearschiebern und die Anordnung der drei Linearschieber in Bezug zu der Taumelscheibe so aufeinander abgestimmt, dass drei unterschiedlich orientierte Schwenkachsen C₁, C₂, C₃ durch jeden der drei Kopfabschnitte der drei Hebelelemente mit dem Zentrum der Taumelscheibe definiert werden. In einer bevorzugten Ausführungsform sind die drei Kopfabschnitte der drei Hebelelemente möglichst in gleichem Abstand zum Zentrum der Taumelscheibe und möglichst gleichmäßig voneinander beabstandet angeordnet, sodass die drei Schwenkachsen C₁, C₂, C₃ hierbei jeweils einen Winkel von etwa 120° ± 10° zueinander aufweisen und in Summe 360° ergeben, wenn sich die Taumelscheibe in Neutralstellung befindet.

In dieser Ausführungsform liegen die Kopfabschnitte der drei Hebelelemente in den Ecken eines gleichseitigen Dreiecks, wobei die Schwenkachsen C₁, C₂, C₃ den Mittelsenkrechten des gleichseitigen Dreiecks entsprechen. Dabei entspricht das Zentrum der Taumelscheibe, um das die Taumelscheibe verschwenkt wird, dem Schnittpunkt der Mittelsenkreckten in diesem gleichseitigen Dreieck. Da in dieser Ausführungsform die Schwenkebene bereits durch die drei Kopfabschnitte definiert ist, verkörpert diese Anordnung gleichzeitig die kardanische Lagerung der Taumelscheibe, sodass zusätzliche Elemente für eine kardanische Lagerung der Taumelscheibe hier entfallen können. Allerdings sind auch alternative Ausführungsformen zu dieser bevorzugten Anordnung denkbar, wenn z. B. die Bauraumgestaltung keine gleichmäßig verteilte Anordnung der drei Kopfabschnitte zulässt. In alternativen Anordnungen, die ebenfalls funktionsfähig sind, können die Kopfabschnitte der Hebelelemente daher auch in den Ecken eines beliebigen gleichschenkligen oder unregelmäßigen Dreiecks liegen, wobei der Schnittpunkt der Mittelsenkrechten des Dreiecks dem Zentrum der Taumelscheibe entspricht.

Somit kann zwischen zwei der drei Schwenkachsen auch ein rechter oder spitzer Winkel vorliegen, sodass zumindest eine oder jede der zwei Schwenkachsen mit der dritten Schwenkachse einen stumpfen Winkel aufspannt. Wenn beispielsweise ein rechter oder spitzer Winkel zwischen zwei der Schwenkachsen im Bereich von 10° und 90° liegt, ergibt sich, im Falle einer gleichschenkligen Anordnung der Kopfabschnitte, ein stumpfer Winkel zwischen jeder der zwei Schenkwachsen und der dritten Schwenkachse im Bereich von 135° bis 175° vor, sodass die Winkel in Summe 360° ergeben. Die Winkelmaße sind nur beispielhaft zu verstehen und sollen den Schutzumfang nicht beschränken. Ein minimaler spitzer Winkel zwischen zwei der drei Schwenkachsen ergibt sich, wenn zwei der drei Kopfabschnitte nebeneinander an dem Umfangsabsatz angeordnet werden, und hängt demnach von dem Durchmesser der Kopfabschnitte und deren Abstand zum Zentrum der Taumelscheibe ab. Grundsätzlich ist auch denkbar, dass zwei der drei Schwenkachsen einen überstumpfen Winkel (> 180°) aufspannen, sodass zumindest eine dieser zwei Schwenkachsen mit der dritten Schwenkachse einen spitzen Winkel aufspannt.

Gemäß einer noch weiteren alternativen Ausführungsform des erfindungsgemäßen Lenkgetriebes, wobei die Taumelscheibe ebenfalls mit dem umlaufenden Umfangsabsatz als Eingriffsöffnung ausgebildet ist, weist das Lenkgetriebe vier Linearschieber mit jeweils einem Hebelelement auf. Hierbei sind die Anordnung der Hebelelemente an den Linearschiebern und die Anordnung der vier Linearschieber in Bezug zu der Taumelscheibe so aufeinander abgestimmt, dass jeweils zwei der Hebelelemente gegenüberliegend angeordnet sind. Dann verlaufen zwei Schwenkachsen C₁, C₂, die durch die Kopfabschnitte gegenüberliegender Hebelelemente mit dem Zentrum der Taumelscheibe definiert werden, orthogonal zueinander. Dabei sind zwei Antriebe mit den Linearschiebern gegenüberliegender Hebelelemente paarweise verbunden. Dies ist ausreichend, da die Linearschieber gegenüberliegender Hebelelemente zur Ausrichtung der Taumelscheibe exakt gegenläufig vom gleichen Antrieb über ein Umkehrgetriebe angesteuert werden. Grundsätzlich ist es allerdings bei ausreichendem Bauraum alternativ auch möglich, für jeden der vier Linearschieber einen eigenen Antrieb einzusetzen.

Gemäß noch einer weiteren alternativen Ausführungsform des erfindungsgemäßen Lenkgetriebes ist vorgesehen, dass an der Taumelscheibe zumindest zwei der Hebelelemente angeordnet sind, wobei die zugehörigen Eingriffsöffnungen an zumindest zwei Linearschiebern vorliegen. Die Hebelelemente, die mit dem Kugelkopfabschnitt ausgebildet sind, erstrecken sich von dem Umfangsrand der Taumelscheibe in radialer Richtung. Und von den zumindest zwei Linearschiebern weist ein erster Linearschieber eine um die Hauptachse A verlaufende Kreisbogennut als Eingriffsöffnung in dem der Taumelscheibe zugewandten Oberflächenabschnitt auf. In der Kreisbogennut des ersten Linearschiebers ist der Kugelkopfabschnitt eines ersten der Hebelelemente beweglich aufgenommen. Ein zweiter Linearschieber weist eine zylindrische Bohrung als Eingriffsöffnung auf, die in dem der Taumelscheibe zugewandten Oberflächenabschnitt ausgebildet ist und in der der Kugelkopfabschnitt eines zweiten der Hebelelemente beweglich aufgenommen ist. Die Achse der zylindrischen Bohrung im zweiten Linearschieber schneidet dabei orthogonal die Hauptachse A. In dieser Bohrung kann sich der Kugelkopfabschnitt des zweiten Hebelelements linear bewegen und den beim Verschwenken der Taumelscheibe variierenden Abstand zwischen Hebelelement und Linearschieber zu kompensieren. Hierbei sind die Anordnung der Eingriffsöffnungen an den Linearschiebern und die Anordnung der zumindest zwei Linearschieber in Bezug zu der Taumelscheibe auf die Anordnung der zumindest zwei Hebelelemente an der Taumelscheibe abgestimmt. Diese weist hierbei einen Zentralabschnitt auf, der mit der Hauptwelle rotativ gekoppelt und drehbar in einem Lenkring der Taumelscheibe gelagert ist, der den Umfangsrand aufweist, an dem die zumindest zwei Hebelelemente angeordnet sind.

Auf diese Weise kann der Zentralabschnitt der Taumelscheibe, an dem die Lenkdrähte befestigt sind, mit der Hauptwelle rotiert werden, während die räumliche Ausrichtung der Taumelscheibe über den Lenkring erfolgt, der infolge des Eingriffs der Hebelelemente mit den Linearschiebern nicht rotierbar ist. Dies liegt an dem Eingriff des Kugel-Kopfabschnitts des zweiten Hebelelements in der Zylinderbohrung des zweiten Linearschiebers, während der Kopfabschnitt des ersten Hebelelements in der Kreisbogennut des ersten Linearschiebers geführt wird. Als Kreisbogennut wird eine beidseitig begrenzte Nut mit der Form eines Kreisbogens verstanden, dessen Kreismittelpunkt auf der Hauptachse A liegt. Die Kreisbogennut verläuft damit quasi parallel zu dem Umfangsrand der Taumelscheibe, wenn diese sich in neutraler Stellung orthogonal zur Hauptachse A befindet.

Beispielsweise kann das Lenkgetriebe in dieser Ausführungsform genau zwei Hebelelemente an dem Umfangsrand der Taumelscheibe aufweisen, die mit dem ersten und zweiten Linearschieber in Eingriff stehen. Dabei sind das erste und das zweite Hebelelement, deren Kugelkopfabschnitte mit dem Zentrum der Taumelscheibe zwei Schwenkachsen C₁, C₂ definieren, so an dem Umfangsrand der Taumelscheibe angeordnet, dass die beiden Schwenkachsen C₁, C₂ unterschiedlich orientiert sind, d. h., einen Winkel aufspannen, der weder 0° noch 180° beträgt. Es ist günstig, wenn der Winkel zwischen den beiden Schwenkachsen C₁, C₂ in einem Bereich von 60° bis 120°liegt. Besonders bevorzugt ist eine orthogonale Anordnung der zwei Hebelelemente am Umfangsrand der Taumelscheibe, d. h., dass die hier den Schwenkachsen C₁, C₂ entsprechenden Längsachsen der Stababschnitte des ersten und zweiten Hebelelements orthogonal zueinander verlaufen. Entsprechend erfolgt die Anordnung der Linearschieber mit den Eingriffsöffnungen passend zum Eingriff mit den Hebelelementen.

Ferner kann ein erfindungsgemäßes Lenkgetriebe nach einer weiteren Ausführungsform zumindest eine Gehäusekomponente aufweisen, die für jeden Linearschieber eine Linearführung bereitstellt. Beispielsweise kann die Linearführung darin bestehen, dass in der Gehäusekomponente für jeden Linearschieber eine Führungsnut parallel zu der Führungsachse Bₓ ausgebildet ist. Entsprechend weist jeder Linearschieber an einer zweiten zur Führungsachse Bₓ parallelen Längsseite, die sich von der Längsseite mit dem der Taumelscheibe zugewandten Oberflächenabschnitt unterscheidet, zumindest ein Führungselement auf, das in der jeweiligen Führungsnut längsbeweglich aufgenommen ist. Die zweite Längsseite des Linearschiebers, an der das Führungselement ausgebildet ist, kann - je nach Gestaltung der Gehäusekomponente - abgewandt von oder benachbart zu der Längsseite des Linearschiebers mit dem der Taumelscheibe zugewandten Oberflächenabschnitt sein. Alternativ kann zur Längsführung der Linearschieber in umgekehrter Weise in der Gehäusekomponente für jeden Linearschieber ein Führungselement parallel zu der Führungsachse Bₓ ausgebildet sein, und jeder Linearschieber an einer zweiten Längsseite des Linearschiebers, die sich von der Längsseite mit dem der Taumelscheibe zugewandten Oberflächenabschnitt unterscheidet, eine Führungsnut aufweisen, die verschiebbar das Führungselement aufnimmt.

Gemäß einer weiteren Ausführungsform des erfindungsgemäßen Lenkgetriebes ist der der Taumelscheibe zugewandten Oberflächenabschnitt konkav geformt, wobei der konkave Oberflächenabschnitt bevorzugt einem Zylindermantelflächenabschnitt um die Hauptachse A entspricht.

Schließlich ist in einer Weiterbildung des erfindungsgemäßen Lenkgetriebes vorgesehen, dass der Antrieb, mit dem der Linearschieber verbunden ist, ein Linearmotor oder ein Rotationsmotor, der über eine Spindel mit dem Linearschieber verbunden ist, oder ein Hydraulik- oder Pneumatikzylinder ist.

Ein erfindungsgemäßes chirurgisches Instrument weist nach einer ersten Ausführungsform einen Instrumentenschaft, an einem distalen Schaftende ein Werkzeug und an einem proximalen Schaftende eine Handhabe auf, die ein Lenkgetriebe mit zumindest zwei Antrieben zur Ausrichtung einer Taumelscheibe aufweist. Diese Taumelscheibe ist rotativ mit einer um eine Hauptachse A rotierbare Hauptwelle gekoppelt und kardanisch um ein Zentrum Z gelagert, das auf der Hauptachse A liegt. Ferner ist die Taumelscheibe mit einer Mehrzahl Lenkdrähte verbunden, die sich entlang der Hauptachse A durch den Instrumentenschaft zu einer Abwinkelungsmechanik des Werkzeugs erstrecken. Bei dem Lenkgetriebe des erfindungsgemäßen chirurgischen Instruments handelt es sich um ein erfindungsgemäßes Lenkgetriebe nach zumindest einer der vorbeschriebenen Ausführungsformen.

Weitere Ausführungsformen sowie einige der Vorteile, die mit diesen und weiteren Ausführungsformen verbunden sind, werden durch die nachfolgende ausführliche Beschreibung unter Bezug auf die begleitenden Figuren deutlich und besser verständlich. Gegenstände oder Teile derselben, die im Wesentlichen gleich oder ähnlich sind, können mit denselben Bezugszeichen versehen sein. Die Figuren sind lediglich eine schematische Darstellung einer Ausführungsform der Erfindung. Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Dabei zeigen:
- Fig. 1: eine perspektivische Teilansicht eines erfindungsgemäßen chirurgischen Instruments mit schematisiert dargestellter Handhabe,
- Fig. 2: eine perspektivische Ansicht eines Lenkgetriebes gemäß einer bevorzugten erfindungsgemäßen Ausführungsform,
- Fig. 3: eine perspektivische Teilansicht des Lenkgetriebes aus Fig. 2 ohne Darstellung der Taumelscheibe,
- Fig. 4: eine perspektivische Ansicht eines Lenkgetriebes gemäß einer weiteren erfindungsgemäßen Ausführungsform,
- Fig. 5: eine perspektivische Ansicht von Taumelscheibe und Linearschiebern eines Lenkgetriebes gemäß einer weiteren erfindungsgemäßen Ausführungsform,
- Fig. 6: eine perspektivische Ansicht von Taumelscheibe und Linearschiebern eines Lenkgetriebes gemäß einer weiteren erfindungsgemäßen Ausführungsform,
- Fig. 7: eine perspektivische Ansicht von Taumelscheibe und Linearschiebern eines Lenkgetriebes gemäß einer weiteren erfindungsgemäßen Ausführungsform,
- Fig. 8: eine perspektivische Ansicht von Taumelscheibe und Linearschiebern eines Lenkgetriebes gemäß einer weiteren erfindungsgemäßen Ausführungsform.

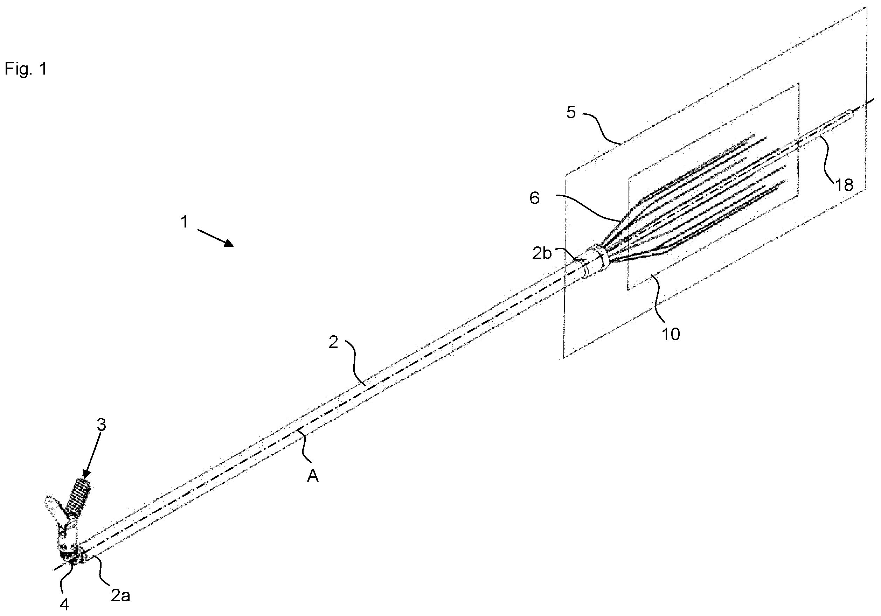

In Fig. 1 ist ein chirurgisches Instrument 1 mit einem hohlen Instrumentenschaft 2 gezeigt, wobei eine am proximalen Ende 2b des Instrumentenschaftes 2 angeordnete Handhabe 5 nur schematisch dargestellt ist. Am distalen Ende 2a des Instrumentenschaftes 2 ist ein Werkzeug 3 angeordnet, bei dem es sich beispielsweise um ein mit Maulteilen versehenes Werkzeug 3, wie in Fig. 1 dargestellt, oder aber um ein Endoskop, einen Applikator oder dergleichen handeln kann. Zur Betätigung des Werkzeugs 3, z. B. zum Öffnen und Schließen der Maulteile, weist das chirurgische Instrument 1 ein axial verschiebbar im Instrumentenschaft 2 gelagertes Betätigungselement 18 auf, das proximalseitig mit einer (nicht dargestellten) Betätigungseinheit der Handhabe 5 in Wirkverbindung steht. Bei der Betätigungseinheit kann es sich um ein manuell betätigbares Griffteil oder um eine für den robotischen Einsatz ausgelegte, also auch ohne manuelles Zutun betätigbare Baueinheit handeln, die mit einem entsprechenden Antrieb gekoppelt ist. Das axialverschiebbar im Instrumentenschaft 2 gelagerte Betätigungselement 18 zum Betätigen des Werkzeugs 3 ist in der Darstellung als Zug-/Schubstange ausgebildet.

Des Weiteren ist das Werkzeug 3 des chirurgischen Instruments 1 über eine Abwinkelungsmechanik 4 an dem distalen Schaftende 2a relativ zur Hauptachse A des Instrumentenschaftes 2 verschwenkbar. Die Abwinkelungsmechanik 4 besteht aus Schwenkgliedern, die über Lenkdrähte 6, die sich durch den Instrumentenschaft 2 erstrecken, mit dem Lenkgetriebe 10 in der Handhabe 5 am proximalen Ende 2b des Instrumentenschaftes 2 verbunden sind. Eine Bewegung des proximalseitigen Lenkgetriebes 10 verursacht so eine durch die Lenkdrähte 6 übertragene entsprechende relative Bewegung der distalseitigen Schwenkglieder der Abwinkelungsmechanik 4 und somit ein Verschwenken des Werkzeugs 3. Auch wenn vorliegend der Begriff Lenkdrähte 6 verwendet wird, können funktional auch Lenkseile verwendet werden, weshalb der verwendete Begriff Lenkdrähte 6 synonym auch als Lenkseil zu lesen und zu verstehen ist.

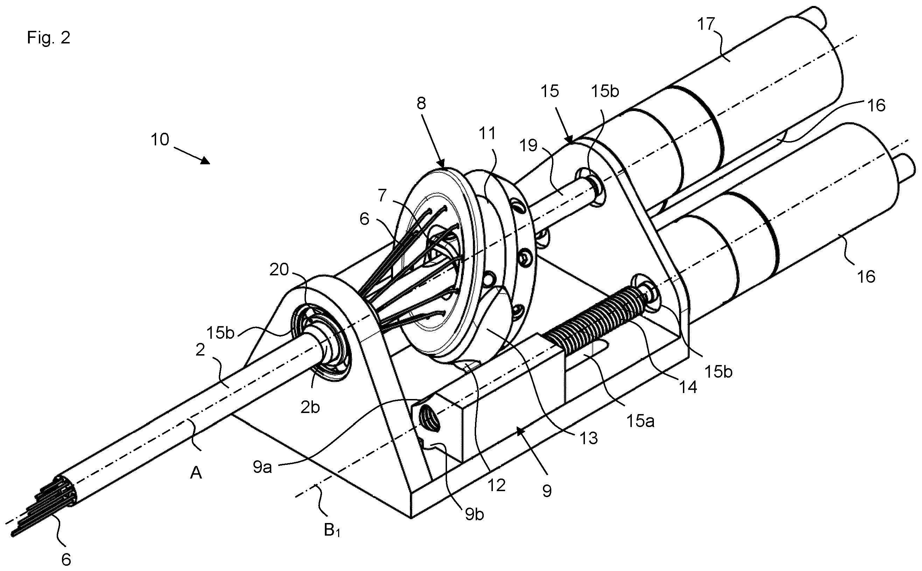

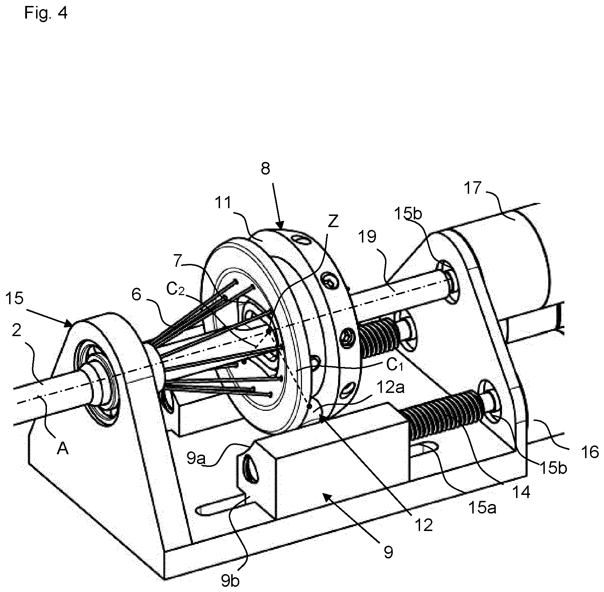

Das Lenkgetriebe 10, für das in **Fig. 2 bis 8** unterschiedliche Beispiele gezeigt sind, weist eine Taumelscheibe 8 auf, an der die aus dem proximalen Schaftende 2b austretenden Lenkdrähte 6 befestigt sind, wie in **Fig. 2** **und** **4** zu sehen ist. Dabei ist die Taumelscheibe 8 zur Rotation mit einer Hauptwelle 19 gekoppelt, die sich entlang der Hauptachse A an das proximale Ende 2b des Instrumentenschafts 2 anschließt. Der Instrumentenschaft 2 ist an seinem proximalen Ende 2b mittels Lager 20 drehbar in einer Durchtrittsöffnung 15b der Gehäusekomponente 15 des Lenkgetriebes 10 gelagert. Die Hauptwelle 19 erstreckt sich proximalseitig durch eine weitere Durchtrittsöffnung 15b in der Gehäusekomponente 15 und steht mit einem Rotationsantrieb 17 in Wirkverbindung. Durch die rotative Kopplung der Hauptwelle 19 mit der Taumelscheibe 8 kann das Werkzeug 3 am distalen Schaftende 2a auch um die Hauptachse A des Instrumentenschaftes 2 rotiert werden, ohne dass die Lenkdrähte 6 verdrillt werden.

Zum Verschwenken um zwei Raumachsen orthogonal zur Hauptachse A ist die Taumelscheibe 8 kardanisch um ein Zentrum Z gelagert, das auf der Hauptachse A liegt. Bei einer üblichen kardanischen Lagerung mit zwei orthogonalen Achspaaren liegt das Zentrum Z im Schnittpunkt der beiden Achspaare und kann dem Taumelscheibenmittelpunkt bzw. Taumelscheibenschwerpunkt entsprechen.

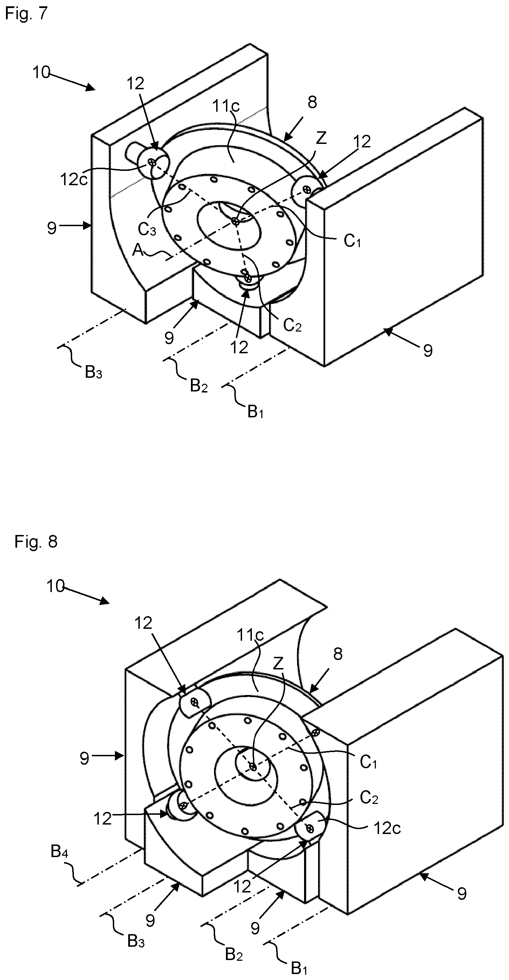

Um die kardanisch gelagerte Taumelscheibe 8 räumlich um ihr Zentrum Z zu verschwenken, weist das Lenkgetriebe 10 je nach Ausführungsbeispiel zwei Linearschieber 9 **(****Fig. 2 bis 6****)** oder drei oder vier Linearschieber 9 **(****Fig. 7, 8****)** auf, die mit der Taumelscheibe 8 in Wirkverbindung stehen. Die Linearschieber 9 sind dabei mit Antrieben 16 verbunden, sodass die Lenkdrähte 6 zum Verschwenken der distalseitigen Schwenkglieder der Abwinkelungsmechanik 4 bzw. des Werkzeugs 3 exakt, feinfühlig in kleinsten Schritten und auch reproduzierbar angesteuert werden können.

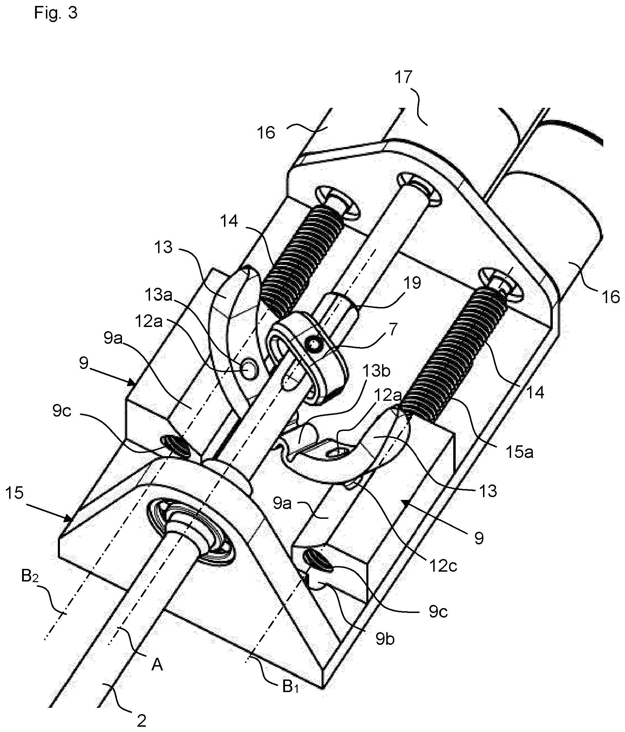

In dem in **Fig. 2 bis 4** gezeigten Beispiel hat die Taumelscheibe 8 eine zentrale, zur Hauptachse A konzentrische Durchtrittsöffnung (unbezeichnet), in der eine Kreuzgelenkscheibe 7 angeordnet ist, die wiederum eine Durchtrittsöffnung (unbezeichnet) aufweist, durch die sich die Hauptwelle 19 erstreckt. Die Kreuzgelenkscheibe 7 ist über ein erstes Achspaar mit der Taumelscheibe 8 schwenkbar verbunden. Über ein zweites Achspaar, das orthogonal zu dem ersten Achspaar angeordnet ist, ist die Kreuzgelenkscheibe 7 schwenkbar mit der Hauptwelle 19 verbunden, wie gut in der Darstellung von Fig. 3 ohne Taumelscheibe zu sehen ist. Der Schnittpunkt der beiden Achspaare, der das Zentrum Z der Taumelscheibe definiert, befindet sich hierbei im Mittelpunkt der Durchtrittsöffnung der Kreuzgelenkscheibe 7, der auch dem Mittelpunkt der Durchtrittsöffnung der Taumelscheibe 8 entspricht. Dabei sorgt die kardanische Lagerung mit der Kreuzgelenkscheibe 7 über die beiden Achspaare auch für die rotative Kopplung der Taumelscheibe 8 mit der Hauptwelle 19.

Nicht dargestellt sind alternative Ausführungen der kardanischen Lagerung der Taumelscheibe, die beispielsweise auch als außenliegende kardanische Lagerung mit einem äußeren Bügel (anstelle eines geschlossenen Rings) ausgeführt sein kann, der im Bereich der Linearschieber ausgespart ist und über ein erstes Achspaar mit einem inneren Ring verbunden ist. Dieser ist über ein zum ersten Achspaar orthogonales zweites Achspaar mit einem Umfangsabschnitt der Taumelscheibe verbunden. Ein zentraler Taumelscheibenabschnitt, an dem die Lenkdrähte befestigt sind und der zur Rotation mit der Hauptwelle gekoppelt ist, ist dann drehbar in dem Umfangsabschnitt der Taumelscheibe gelagert.

Das Lenkgetriebe 10 der Beispiele in **Fig. 2 bis 6** weist zwei Linearschieber 9 auf, die parallel zu dem Rotationsantrieb 17 der Hauptwelle 19 angeordnet sind. Jeder Linearschieber 9 kann durch die Verbindung mit einem Antrieb 16 unabhängig entlang einer zu der Hauptachse A parallelen Führungsachse B₁, B₂ verschoben werden. Die Antriebe 16, mit denen die Linearschieber 9 betätigt werden, können ebenfalls Rotationsmotoren 16 sein, wie in **Fig. 2** **und** **3** dargestellt. Dazu sind die Rotationsmotoren 16 über jeweils eine Spindel 14 mit dem jeweiligen Linearschieber 9 verbunden, der hierzu eine Spindelbohrung 9c längs der jeweiligen Führungsachse B₁, B₂ aufweist. Alternativ zu einem Rotationsmotor mit Spindel kann als Antrieb 16 beispielsweise ein Linearmotor oder ein Hydraulik- oder Pneumatikzylinder eingesetzt werden.

Um die Bewegung des jeweiligen Linearschiebers 9 auf die Taumelscheibe 8 zu übertragen, um diese um ihr Zentrum Z zu verschwenken, steht jeder Linearschieber 9 mit der Taumelscheibe 8 über ein Eingriffselementepaar in Wirkverbindung. Die Eingriffselemente eines Eingriffselementepaars können, wie nachfolgend beschrieben wird, in unterschiedlicher Weise ausgeführt sein. Aber jedes Eingriffselementepaar weist als eines der Eingriffselemente ein Hebelelement 12 mit einem Kopfabschnitt 12a, 12b auf, der beweglich in einer Eingriffsöffnung 11, 11a, 11b, 11c aufgenommen ist, die das andere Eingriffselement darstellt. Dabei liegt eines der Eingriffselemente des Eingriffselementepaars an einem Umfang der Taumelscheibe 8 vor, und das andere Eingriffselement des Eingriffselementepaars befindet sich an einem Oberflächenabschnitt 9a des jeweiligen Linearschiebers 9, der der Taumelscheibe 8 zugewandt ist. Auf diese Weise befinden sich die Kopfabschnitte 12a, 12b der Hebelelemente 12 immer auf einer Umfangslinie um das Zentrum Z der Taumelscheibe 8.

Der Oberflächenabschnitt 9a, der der Taumelscheibe 8 zugewandt ist und an dem das jeweilige Eingriffselement vorliegt, ist an einer Längsseite des Linearschiebers 9, die parallel zur Führungsachse B₁, B₂ liegt, als konkaver Oberflächenabschnitt 9a ausgebildet. Dabei entspricht der Oberflächenabschnitt 9a einem Zylindermantelflächenabschnitt um die Hauptachse A mit einem Radius, der etwas größer als der Radius der Taumelscheibe 8 ist. Die Schwenkebene der Taumelscheibe 8 wird dabei durch das Zentrum Z der kardanischen Lagerung und die Eingriffspunkte der Kopfabschnitte 12a, 12b am freien Ende der Stababschnitte 12c der der Hebelelemente 9 definiert.

Zur achsparallelen Führung der Linearschieber 9 ist in der Gehäusekomponente 15 parallel zu jeder Führungsachse B₁, B₂ jeweils eine Führungsnut 15a ausgebildet, in der ein Führungselement 9b des jeweiligen Linearschiebers 9 verschiebbar aufgenommen ist. Das Führungselement 9b ist an der Unterseite des Linearschiebers 9 als längliches Profilelement ausgebildet, das sich in Längsrichtung parallel zur Führungsachse B₁, B₂ erstreckt. In einer von der beispielhaften Darstellung abweichenden Gestaltung der Gehäusekomponente und der Linearschieber ist es alternativ möglich, dass das Führungselement an dem Linearschieber nicht an dessen Unterseite, sondern an anderen angrenzenden Seitenfläche vorliegt, die nicht den der Taumelscheibe 8 zugewandten Oberflächenabschnitt 9a aufweist.

Ferner kann die Anordnung von Führungselement und Führungsnut an Linearschieber und Gehäusekomponente in einer nicht dargestellten Ausführungsform auch umgekehrt sein, d. h., dass der Linearschieber eine Längsnut aufweist und an der Gehäusekomponente in längliches Führungselement vorliegt, auf dem der Linearschieber mit der Längsnut verschiebbar ist. Abweichend zur Kombination aus Führungselement und Führungsnut kann die Linearführung der Linearschieber eines erfindungsgemäßen Lenkgetriebes auch andere, bekannte Linearführungselemente wie z. B. C-Profile oder Wellen umfassen.

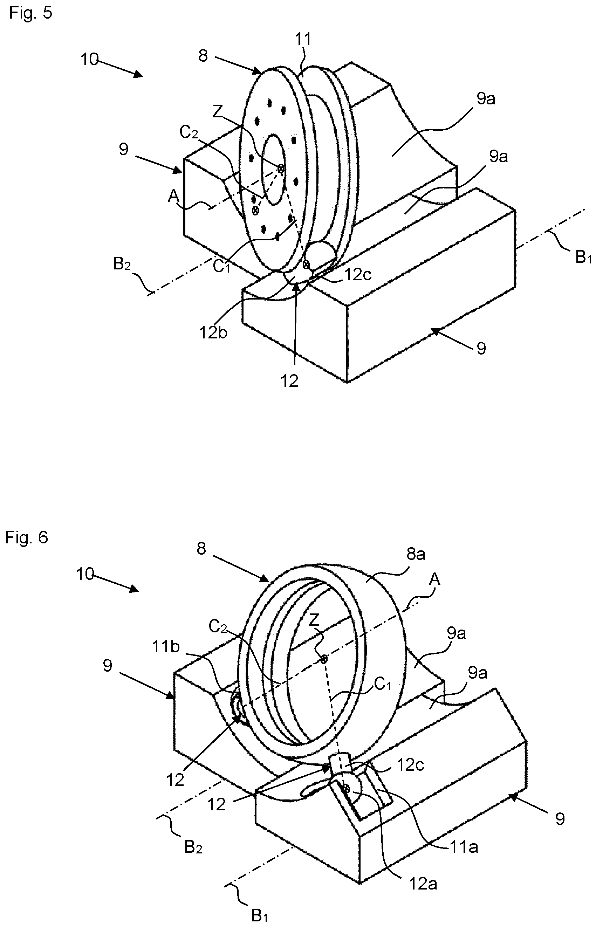

Abgesehen vom Beispiel in **Fig. 6** sind in den Lenkgetrieben 10 die Hebelelemente 12 an den Linearschiebern 9 angeordnet, während die zugehörige Eingriffsöffnung an der Taumelscheibe 8 ausgebildet ist, und zwar in Form einer entlang des Umfangrands der Taumelscheibe 8 umlaufenden Umfangsnut 11 **(****Fig. 2****,** **4****,** **5****)** oder in Form eines entlang des Umfangrands der Taumelscheibe 8 umlaufenden Umfangsabsatzes 11c **(****Fig. 7, 8****).** Dabei bildet die umlaufende Umfangsnut 11 bzw. der umlaufende Umfangsabsatz 11c ein gemeinsames Eingriffselement für alle Hebelelemente 12. Jedes Hebelelement 12 ist an dem konkaven Oberflächenabschnitt 9a des jeweiligen Linearschiebers 9 angeordnet, sodass der Stababschnitt 12c in radialer Richtung zur Hauptachse A weist und damit der Kopfabschnitt 12a, 12b in der Umfangsnut 11 aufgenommen werden bzw. an dem Umfangsabsatz 11c zur Anlage kommen kann.

Auf diese Weise liegen die Mittelpunkte der Kopfabschnitte 12a, 12b durch die Führung in der Umfangsnut 11 bzw. an dem Umfangsabsatz 11c auf einer Umfangslinie um das Taumelscheibenzentrum Z und damit stets exakt in der Schwenkebene der Taumelscheibe 8. Werden die Linearschieber 9 und damit die Hebelelemente 12 entlang der jeweiligen Führungsachse B₁, B₂ parallel zur Hauptachse A bewegt, verschieben sich diese Mittelpunkte der Kopfabschnitte 12a, 12b entsprechend und führen so zur Verschwenkung der Taumelscheibe 8. Weil die Umfangsnut 11 bzw. der Umfangsabsatz 11c umlaufend ist, kann die Taumelscheibe 8 mit der Hauptwelle 19 trotzdem frei rotiert werden. So kann die Taumelscheibe 8 einerseits in zwei Richtungen gegenüber der Hauptachse A verschwenkt und die befestigten Lenkdrähte 6 unterschiedlich ausgelenkt werden und andererseits gemeinsam mit der Hauptwelle 19 rotiert werden, sodass das Werkzeug 3 des chirurgischen Instruments 1 aus **Fig. 1** am distalen Ende 2a des Instrumentenschafts 2 entsprechend abgewinkelt und/oder um die Hauptachse A rotiert werden kann.

In Fig. 4 ist die Taumelscheibe 8 in neutraler Stellung gezeigt, in der die Hauptachse A orthogonal zu der Schwenkebene verläuft, die durch das Zentrum Z der Taumelscheibe 8 und die Kugelkopfabschnitte 12a definiert wird, die in der Umfangsnut 11 der Taumelscheibe 8 aufgenommen und beidseitig geführt sind. Die Anordnung der zwei Linearschieber 9 in Bezug zu der Taumelscheibe 8 und die Anordnung der Hebelelemente 12 an den zwei Linearschiebern 9 sind dabei in dem gezeigten Beispiel so aufeinander abgestimmt, dass zwei Schwenkachsen C₁, C₂, die durch die beiden Kugelkopfabschnitte 12a der beiden Hebelelemente 12 mit dem Zentrum Z der Taumelscheibe 8 definiert werden, in der Neutralstellung der Taumelscheibe 8 orthogonal zueinander verlaufen. Der Kugelkopfabschnitt 12a weist annähernd Kugelform auf, die allerdings, wie in den Beispielen von **Fig. 3****,** **7 und 8** zu sehen ist, an der zur Taumelscheibe 8 weisenden Seite abgeflacht sein kann.

Die Lage der Schwenkebene der Taumelscheibe 8, die durch die beiden Schwenkachsen C₁, C₂ definiert ist, wird durch die Position der Linearschieber 9 bestimmt, wobei das Verschwenken der Taumelscheibe 8 in Bezug auf die Hauptachse 8 durch die kardanische Lagerung ermöglicht wird. Durch Verschieben eines oder beider Linearschieber 9 entlang der jeweiligen Führungsachse B₁, B₂ wird die Taumelscheibe 8 aus der neutralen Stellung verschwenkt, wie z. B. in **Fig. 2** **oder** **5** zu sehen ist, die Ausführungsbeispiele zeigen, die dem in **Fig. 4** ähnlich sind. Das Lenkgetriebe 10 in **Fig. 2** unterscheidet sich von **Fig. 4** durch die Anordnung zweier Nutensteinelemente 13, die die Kraftübertragung der Hebelelemente 12 auf die Taumelscheibe 8 verbessern, indem die Kontaktfläche gegenüber dem Kugelkopfabschnitt 12a vergrößert wird. Die Nutensteinelemente 13 sind zur führbaren Aufnahme in der Umfangsnut 11 ausgebildet und weisen eine Kugelpfanne 13a zur beweglichen Aufnahme des Kugelkopfabschnitts 12a auf (vgl. Darstellung ohne Taumelscheibe in **Fig. 3****).** Die Nutensteinelemente 13 übertragen die Lenkkräfte der in den Kugelpfannen 13a aufgenommenen Kugelkopfabschnitte 12a flächig auf die Taumelscheibe 8 durch die Anordnung in der Umfangsnut 11. Da der Abstand zwischen beiden Kugelkopfabschnitte 12a bei Betätigung der Linearschieber 9 variiert, sind die beiden Nutensteinelemente 13 im gezeigten Beispiel über ein flexibles Verbindungsstück 13 b miteinander verbunden, um eine Art Klammerelement zu bilden. Alternativ können die Nutensteinelemente 13 unabhängig voneinander als einzelne Nutensteine 13 ausgeführt sein.

Der Unterschied zwischen den Lenkgetrieben 10 in **Fig. 4** **und** **5** besteht darin, dass die Hebelelemente 12 in **Fig. 5** mit einem T-Kopfabschnitt 12b anstelle einem Kugelkopfabschnitt 12a wie in **Fig. 4** ausgebildet sind. Der T-Kopfabschnitt 12b weist eine zylinderartige Form auf, die einem Torusabschnitt ähnelt, d. h. quasi einem entlang der Zylinderachse gebogenen Zylinder, dessen Biegeradius mit dem Umfang der Taumelscheibe 8 in der Umfangsnut 11 korrespondiert. Dabei weist der T-Kopfabschnitt 12b im Querschnitt eine Kreissegmentform auf, die zu einer abgeflachten Seite der Mantelfläche führt, an der der T-Kopfabschnitt 12b an dem Stababschnitt 9c angeordnet ist und der zu dem konkaven Oberflächenabschnitt 9a des Linearschiebers 9 weist. Mit der gerundeten Mantelfläche berührt der T-Kopfabschnitt 12b die Taumelscheibe 8 in der Umfangsnut 11 somit entlang einer Linie. Damit sich die T-Kopfabschnitte 12b in der Umfangsnut 11 beim Verschwenken der Taumelscheibe 8 durch den jeweils anderen Linearschieber 9 ausrichten können, sind die Hebelelemente 12, die den T-Kopfabschnitt 12b aufweisen, drehbar um die Längsachse des Stababschnitts 12c in dem Linearschieber 9 gelagert.

Die alternativen Ausführungen der Lenkgetriebe 10 in **Fig. 7 und 8** verwenden als Eingriffsöffnung an der Taumelscheibe 8 einen Umfangsabsatz 11c, der einen nur einseitig wirkenden Kontakt zum Kugelkopfabschnitt 12a des Hebelelements 12 bereitstellt. Daher sind drei Linearschieber 9 mit drei Hebelelementen 12 **(****Fig. 7****)** oder vier Linearschieber 9 mit vier Hebelelementen 12 **(****Fig. 8****)** notwendig, um alle räumlichen Lagen der Taumelscheibe 8 zu definieren. Der Umfangsabsatz 11c weist ähnlich einem Ringflansch einen distalseitigen Zylinderabschnitt und einen davon abgesetzten proximalseitigen Scheibenabschnitt mit vergrößertem Durchmesser auf. Die Kugelkopfabschnitte 12a der Hebelelement 12 liegen daher distalseitig an dem Scheibenabschnitt des Umfangsabsatzes 11c an. Um den Eingriff der Hebelelemente 12 an dem so einseitig begrenzten Umfangsabsatz 11c zu sichern, können die Lenkdrähte 6, die an der Stirnseite des distalseitigen Zylinderabschnitts befestigt sind, im Betrieb durch einen Spannmechanismus definiert gespannt werden. Hierdurch kann das Zentrum Z der Taumelscheibe 8 linear entlang der Hauptachse A verschoben werden, sodass die Taumelscheibe 8 mittels der Lenkdrähte 6 in distale Richtung gezogen wird, damit der Umfangsabsatz 11c an den Kopfabschnitten 12a anliegt.

In der Variante von **Fig. 7** mit drei Linearschiebern 9 sind drei Antrieben (nicht dargestellt) vorgesehen, die mit den Linearschiebern 9 verbunden sind, um jeden Linearschieber 9 unabhängig entlang der drei Führungsachsen B₁, B₂, B₃ zur Ausrichtung der Taumelscheibe 8 bewegen zu können. Die Anordnung der Hebelelemente 12 an den jeweiligen Linearschiebern 9 sowie die Anordnung der drei Linearschieber 9 in Bezug zu der Taumelscheibe 8 sind dabei so aufeinander abgestimmt, dass die drei Kopfabschnitte 12a mit dem Zentrum Z der Taumelscheibe drei Schwenkachsen C₁, C₂, C₃ definieren, die jeweils einen Winkel von 120° zueinander aufweisen. Hierbei ergibt sich das Zentrum Z der Taumelscheibe 8 als Mittelpunkt der Kreislinie, auf der die drei Kopfabschnitte 12a gleichmäßig beabstandet angeordnet sind. Da die Lage der Taumelscheibe 8 in Bezug auf zwei Raumrichtungen orthogonal zu der Hauptachse A durch die drei Kopfabschnitte 12a eindeutig bestimmt und damit die kardanische Lagerung im Sinne der Erfindung bereitgestellt ist, kann auf kardanische Lagerungselemente wie die Kreuzgelenkscheibe 7 mit den zwei orthogonalen Achspaaren des Beispiels in **Fig. 2 bis 4** verzichtet werden. Allerdings ist dann für die rotative Kopplung der Taumelscheibe 8 mit der Hauptwelle 19 (nicht dargestellt in **Fig. 7****)** ein alternatives Lagerungskonzept erforderlich, das einerseits die Übertragung von Drehwinkeln der Hauptwelle auf die Taumelscheibe 8 gestattet, ohne das Verschwenken der Taumelscheibe 8 gegenüber der Hauptachse A zu hindern.

Nach einem solchen alternativen Lagerungskonzept ist vorgesehen, dass die Taumelscheibe in ihrer Durchtrittsöffnung einen radial nach innen weisenden Stift oder ein diametral nach innen weisendes Stiftpaar aufweist und in der Hauptwelle entsprechend eine Führungsnut oder zwei diametrale Führungsnuten ausgebildet sind, die sich in Längsrichtung der Hauptwelle erstrecken. Der Eingriff des Stifts/der Stifte in die Führungsnut(en) bewirkt die rotative Kopplung der Hauptwelle mit der Taumelscheibe und gestattet gleichzeitig ein Verschwenken der Taumelscheibe in zwei orthogonalen Raumrichtungen: Ein Verschwenken um die Achse des Stifts/Stiftpaares, da sich jeder Stift in der jeweiligen Führungsnut um seine Achse drehen kann, und ein Verkippen des Stifts in einer Ebene, die er mit der Führungsnut aufspannt, was einem Verschwenken der Taumelscheibe um eine Achse entspricht, die orthogonal zur Stiftachse und zur Hauptachse A verläuft.

Um auf einen dritten Antrieb zur Ausrichtung der Taumelscheibe 8 verzichten zu können, werden im Beispiel von **Fig. 8** vier Linearschiebern 9 mit vier Hebelelementen 9 eingesetzt. Dieses Lenkgetriebe 10 sieht entsprechend vier Führungsachsen B₁, B₂, B₃, B₄ vor, entlang derer die vier Linearschieber 9 zur Ausrichtung der Taumelscheibe 8 bewegen werden können. Hierbei sind die Anordnung der Hebelelemente 12 an den vier Linearschiebern 9 und die Anordnung der vier Linearschieber 9 in Bezug zu der Taumelscheibe 8 so aufeinander abgestimmt, dass die Kopfabschnitte 12a auf einer Kreislinie um das Zentrum Z der Taumelscheibe 8 gleichmäßig beabstandet angeordnet sind, sodass jeweils zwei Kopfabschnitte 12a diametral gegenüberliegend angeordnet sind. Entsprechend definieren die zwei Kopfabschnitte 12a gegenüberliegender Hebelelemente 12 jeweils eine Schwenkachse C₁, C₂, die in Neutralstellung der Taumelscheibe 8 orthogonal zueinander verlaufen und sich im Zentrum Z der Taumelscheibe 8 schneiden. Die Linearschieber 9 gegenüberliegender Hebelelemente 12 werden hierbei nicht unabhängig voneinander, sondern exakt gegenläufig angesteuert, wie dies in **Fig. 8** anhand der Lage der Linearschieber 9 entlang der Führungsachse B₁, B₂, B₃, B₄ zu sehen ist. Der entlang der ersten Führungsachse B₁ geführte Linearschieber 9 (dessen Hebelelement in der Darstellung verdeckt ist) ist in proximale Richtung versetzt, korrespondierend zu dem Versatz in distaler Richtung des entlang der dritten Führungsachse B₃ geführten Linearschiebers 9. Entsprechendes gilt für die entlang der Führungsachsen B₂ und B₄ geführten Linearschieber 9. Die gegenläufige Ansteuerung der Linearschieber 9 gegenüberliegender Hebelelemente 12 kann mit einem nicht dargestellten Umkehrgetriebe (z. B. unter Verwendung einer gegenläufigen Spindel etc.) realisiert werden, sodass sich die Linearschieber 9 gegenüberliegender Hebelelemente 12 jeweils vom gleichen Antrieb bewegen lassen, und daher nur zwei Antriebe für vier Linearschieber erforderlich sind.

Bei dem in **Fig. 6** dargestellten Beispiel eines alternativen Lenkgetriebes 10 sind die Hebelelemente 12 an der Taumelscheibe 8 angeordnet. Die entsprechenden Eingriffsöffnung 11a, 11b für die Kopfabschnitten 12a liegen an den Linearschiebern 9 vor. Dabei ist in einem entlang der ersten Führungsachse B₁ geführten Linearschieber 9 in dem konkaven Oberflächenabschnitt 9a eine um die Hauptachse A verlaufende Kreisbogennut 11a ausgebildet, in der der Kugelkopfabschnitt 12a eines der Hebelelemente 12 führbar aufgenommen ist. Der andere, entlang der zweiten Führungsachse B₂ geführte Linearschieber 9 weist in dem konkaven Oberflächenabschnitt 9a eine Zylinderbohrung 11b auf, deren Achse die Hauptachse A senkrecht schneidet und in der der Kugelkopfabschnitt 12a des zweiten Hebelelements 12 beweglich aufgenommen ist. Da die Hebelelemente 12 in dieser Anordnung eine Rotation der Taumelscheibe 8 um die Hauptachse A verhindern würden, ist die Taumelscheibe 8 in dieser Ausführungsform in einem Umfangsabschnitt 8a, an dem die Hebelelemente 12 angeordnet sind, und einen nicht dargestellten Zentralabschnitt geteilt. Dieser Zentralabschnitt ist drehbar in dem Umfangsabschnitt 8a gelagert und zur Befestigung der Lenkdrähte vorgesehen. Die kardanische Lagerung des Zentralabschnitts auf der Hauptwelle und deren rotative Kopplung kann mittels einer Kreuzgelenkscheibe und zwei gekreuzter Achspaare wie oben beschrieben erfolgen, die das Zentrum Z der Taumelscheibe 8 bestimmen. Die Schwenkachsen C₁, C₂, die durch die Kugelkopfabschnitte 12a der Hebelelemente 12 mit dem Zentrum Z der Taumelscheibe 8 definiert werden, verlaufen orthogonal zueinander. Die Hebelelemente 12 erstrecken sich hierbei entsprechend um 90° versetzt in radialer Richtung von dem Umfangsrand des Umfangsabschnitts 8a der Taumelscheibe 8.

Die Zeichnungen, die Beschreibung und die Ansprüche enthalten zahlreiche Merkmale in Kombination. Es versteht sich, dass die vorstehend genannten Merkmale nicht nur in der jeweils angegebenen Kombination, sondern auch in anderen Kombinationen oder in Alleinstellung verwendbar sind, ohne den Rahmen der vorliegenden Erfindung zu verlassen. Die vorliegende Erfindung stellt ein Lenkgetriebe 10 für ein chirurgisches Instrument 1 und das chirurgische Instrument 1 selbst bereit, wobei das Lenkgetriebe 10 zur räumlichen Ausrichtung einer Taumelscheibe 8 zumindest zwei Antriebe 16 aufweist. Die Taumelscheibe 8 ist mit einer Hauptwelle 19, die um eine Hauptachse A rotierbar ist, rotativ gekoppelt und kardanisch um ein Zentrum Z gelagert, das auf der Hauptachse A liegt. Das Lenkgetriebe 10 weist zumindest zwei Linearschieber 9 auf, die jeweils entlang einer zu der Hauptachse A parallelen Führungsachse Bₓ verschiebbar angeordnet und mit den zumindest zwei Antrieben 16 verbunden sind. Jeder Linearschieber 9 steht mit der Taumelscheibe 8 über ein Eingriffselementepaar in Wirkverbindung, das als Eingriffselemente eine Eingriffsöffnung 11, 11a, 11b, 11c und ein Hebelelement 12 aufweist, das an einem freien Ende eines Stababschnitts 12c einen Kopfabschnitt 12a, 12b zur beweglichen Aufnahme in der Eingriffsöffnung 11, 11a, 11b aufweist, wobei eine Schwenkebene der Taumelscheibe 8 durch die Kopfabschnitte 12a, 12b der Hebelelemente 12 und das Zentrum Z der Taumelscheibe 8 definiert wird. Dabei liegt eines der Eingriffselemente des Eingriffselementepaars an einem Umfang der Taumelscheibe 8 und das andere Eingriffselement des Eingriffselementepaars an einem der Taumelscheibe 8 zugewandten Oberflächenabschnitt 9a des jeweiligen Linearschiebers 9 vor, der ein Oberflächenabschnitt 9a an einer Längsseite des Linearschiebers 9 parallel zur Führungsachse Bₓ ist.

### BEZUGSZEICHENLISTE

- 1: Chirurgisches Instrument
- 2: Instrumentenschaft
- 2a, 2b: Distales, proximales Schaftende
- 3: Werkzeug
- 4: Abwinkelungsmechanik
- 5: Handhabe
- 6: Lenkdraht
- 7: Kreuzgelenkscheibe
- 8: Taumelscheibe
- 8a: Lenkring
- 9: Linearschieber
- 9a, 9b, 9c: Konkaver Oberflächenabschnitt, Führungselement, Spindelbohrung
- 10: Lenkgetriebe
- 11: Umfangsnut
- 11a, 11b: Kreisbogennut, Zylinderbohrung
- 11c: Umfangsabsatz
- 12: Hebelelement
- 12a, 12b, 12c: Kugelkopfabschnitt, T-Kopfabschnitt, Stababschnitt
- 13: Nutensteinelement
- 13a, 13b: Kugelpfanne, Verbindungsstück
- 14: Schieber-Spindel
- 15: Gehäusekomponente
- 15a, 15b: Führungsnut, Durchtrittsöffnung
- 16: Linearschieber-Antrieb
- 17: Rotationsantrieb
- 18: Zugstange
- 19: Hauptwelle
- 20: Lager
- A: Hauptachse
- B_{1, 2, 3, 4}: Führungsachse
- C_{1, 2, 3}: Schwenkachse
- Z: Taumelscheibenzentrum

## Patentansprüche

1. Lenkgetriebe (10) für ein chirurgisches Instrument (1), wobei das Lenkgetriebe (10) zur räumlichen Ausrichtung einer Taumelscheibe (8) zumindest zwei Antriebe (16) aufweist, wobei die Taumelscheibe (8) mit einer Hauptwelle (19), die um eine Hauptachse (A) rotierbar ist, rotativ gekoppelt und kardanisch um ein Zentrum (Z) gelagert ist, das auf der Hauptachse (A) liegt,
**dadurch gekennzeichnet, dass**
das Lenkgetriebe (10) zumindest zwei Linearschieber (9) aufweist, die jeweils entlang einer zu der Hauptachse (A) parallelen Führungsachse (Bₓ) verschiebbar angeordnet und mit den zumindest zwei Antrieben (16) verbunden sind,
wobei jeder Linearschieber (9) mit der Taumelscheibe (8) über ein Eingriffselementepaar in Wirkverbindung steht, das als Eingriffselemente eine Eingriffsöffnung (11, 11a, 11b, 11c) und ein Hebelelement (12) aufweist, das an einem freien Ende eines Stababschnitts (12c) einen Kopfabschnitt (12a, 12b) zur beweglichen Aufnahme in der Eingriffsöffnung (11, 11a, 11b) aufweist, wobei eine Schwenkebene der Taumelscheibe (8) durch die Kopfabschnitte (12a, 12b) der Hebelelemente (12) und das Zentrum (Z) der Taumelscheibe (8) definiert wird, und wobei eines der Eingriffselemente des Eingriffselementepaars an einem Umfang der Taumelscheibe (8) und das andere Eingriffselement des Eingriffselementepaars an einem der Taumelscheibe (8) zugewandten Oberflächenabschnitt (9a) des jeweiligen Linearschiebers (9) vorliegen, der ein Oberflächenabschnitt (9a) an einer Längsseite des Linearschiebers (9) parallel zur Führungsachse (Bₓ) ist.

2. Lenkgetriebe (10) nach Anspruch 1,
**dadurch gekennzeichnet, dass**
der Kopfabschnitt ein Kugelkopfabschnitt (12a), der zumindest teilweise eine Kugelform aufweist, oder ein T-Kopfabschnitt (12b) ist, der zumindest teilweise zylinderartig geformt ist.

3. Lenkgetriebe (10) nach Anspruch 1 oder 2,
**dadurch gekennzeichnet, dass**
die Taumelscheibe (8) die Eingriffsöffnung (11, 11a, 11b, 11c) und jeder Linearschieber (9) jeweils eines der Hebelelemente (12) aufweist,
wobei die Eingriffsöffnung (11, 11a, 11b, 11c) eine entlang des Umfangrands der Taumelscheibe (8) umlaufende Umfangsnut (11) oder ein entlang des Umfangrands der Taumelscheibe (8) umlaufender Umfangsabsatz (11c) ist,
und wobei das Hebelelement (12) an dem der Taumelscheibe (8) zugewandten Oberflächenabschnitt (9a) des jeweiligen Linearschiebers (9) angeordnet ist, sodass der Stababschnitt (12c) in radialer Richtung zur Hauptachse (A) weist,
und wobei der Kopfabschnitt (12a, 12b) jedes Hebelelements (12) in der Umfangsnut (11) oder in dem Umfangsabsatz (11c) beweglich aufgenommen ist.

4. Lenkgetriebe (10) nach Anspruch 3,
**dadurch gekennzeichnet, dass**
das Eingriffselementepaar ferner für jedes Hebelelement (12) mit dem Kugelkopfabschnitt (12a) ein Nutensteinelement (13) aufweist, das zur Aufnahme in der umlaufenden Umfangsnut (11) oder in dem umlaufenden Umfangsabsatz (11c) ausgebildet ist und eine Kugelpfanne (13a) aufweist, die zur beweglichen Aufnahme des Kugelkopfabschnitts (12a) ausgebildet ist.

5. Lenkgetriebe (10) nach Anspruch 3 oder 4,
**dadurch gekennzeichnet, dass**
die Taumelscheibe (8) mit der umlaufenden Umfangsnut (11) als Eingriffsöffnung (11, 11a, 11b, 11c) ausgebildet ist und das Lenkgetriebe (10) zwei Linearschieber (9) aufweist, wobei die Anordnung der Hebelelemente (12) an den zwei Linearschiebern (9) und die Anordnung der zwei Linearschieber (9) in Bezug zu der Taumelscheibe (8) so aufeinander abgestimmt sind, dass zwei unterschiedlich orientierte Schwenkachsen (C₁, C₂) durch die zwei Kopfabschnitte (12a, 12b) der beiden Hebelelemente (12) mit dem Zentrum (Z) der Taumelscheibe (8) definiert werden, wobei die zwei Schwenkachsen (C₁, C₂) bevorzugt orthogonal zueinander verlaufen.

6. Lenkgetriebe (10) nach Anspruch 3 oder 4,
**dadurch gekennzeichnet, dass**
die Taumelscheibe (8) mit dem umlaufenden Umfangsabsatz (11c) als Eingriffsöffnung (11, 11a, 11b, 11c) ausgebildet ist und das Lenkgetriebe (10) drei Linearschieber (9) aufweist, die mit drei Antrieben (16) verbunden sind, wobei die Anordnung der Hebelelemente (12) an den drei Linearschiebern (9) und die Anordnung der drei Linearschieber (9) in Bezug zu der Taumelscheibe (8) so aufeinander abgestimmt sind, dass drei unterschiedlich orientierte Schwenkachsen (C₁, C₂, C₃) durch die drei Kopfabschnitte (12a, 12b) der drei Hebelelemente (12) mit dem Zentrum (Z) der Taumelscheibe (8) definiert werden, wobei die drei Schwenkachsen (C₁, C₂, C₃) bevorzugt jeweils einen Winkel von 120° ± 10° zueinander aufweisen.

7. Lenkgetriebe (10) nach Anspruch 3 oder 4,
**dadurch gekennzeichnet, dass**
die Taumelscheibe (8) mit dem umlaufenden Umfangsabsatz (11c) als Eingriffsöffnung (11, 11a, 11b, 11c) ausgebildet ist und das Lenkgetriebe (10) vier Linearschieber (9) aufweist,
wobei die Anordnung der Hebelelemente (12) an den vier Linearschiebern (9) und die Anordnung der vier Linearschieber (9) in Bezug zu der Taumelscheibe (8) so aufeinander abgestimmt sind, dass zwei Schwenkachsen (C₁, C₂), die durch jeweils zwei der Kopfabschnitte (12a, 12b), deren Hebelelemente (12) gegenüberliegend angeordnet sind, mit dem Zentrum (Z) der Taumelscheibe (8) definiert werden, orthogonal zueinander verlaufen,
wobei die Linearschieber (9) gegenüberliegender Hebelelemente (12) paarweise mit den zwei Antrieben (16) verbunden sind.

8. Lenkgetriebe (10) nach Anspruch 2,
**dadurch gekennzeichnet, dass**
- an der Taumelscheibe (8) zumindest zwei der Hebelelemente (12) angeordnet sind, die sich von dem Umfangsrand der Taumelscheibe (8) in radialer Richtung erstrecken und mit dem Kugelkopfabschnitt (12a) ausgebildet sind, und
- von den zumindest zwei Linearschiebern (9) ein erster Linearschieber (9) eine um die Hauptachse (A) verlaufende Kreisbogennut (11a) als Eingriffsöffnung (11, 11a, 11b, 11c) und ein zweiter Linearschieber eine Zylinderbohrung (11b) als Eingriffsöffnung (11, 11a, 11b, 11c) aufweisen, die in dem der Taumelscheibe (8) zugewandten Oberflächenabschnitt (9a) des jeweiligen Linearschiebers (9) ausgebildet sind,
wobei der Kugelkopfabschnitt (12a) eines ersten der Hebelelemente (12) in der Kreisbogennut (11a) des ersten Linearschiebers (9) und der Kugelkopfabschnitt (12a) eines zweiten der Hebelelemente (12) in der Zylinderbohrung (11b) des zweiten Linearschiebers (9) beweglich aufgenommen sind,
und wobei die Anordnung der Eingriffsöffnungen (11a, 11b) an den Linearschiebern (9) und die Anordnung der zumindest zwei Linearschieber (9) in Bezug zu der Taumelscheibe (8) auf die Anordnung der zumindest zwei Hebelelemente (12) an der Taumelscheibe (8) abgestimmt sind,
und wobei die Taumelscheibe (8) einen Zentralabschnitt aufweist, der mit der Hauptwelle (19) rotativ gekoppelt und drehbar in einem Lenkring der Taumelscheibe (8) gelagert ist, der den Umfangsrand aufweist, an dem die zumindest zwei Hebelelemente (12) angeordnet sind.

9. Lenkgetriebe (10) nach zumindest einem der Ansprüche 1 bis 8,
**dadurch gekennzeichnet, dass**
das Lenkgetriebe (10) zumindest eine Gehäusekomponente (15) aufweist, die für jeden Linearschieber (9) eine Linearführung bereitstellt, und/oder der Oberflächenabschnitt (9a) ein konkaver Oberflächenabschnitt (9a) ist, der bevorzugt einem Zylindermantelflächenabschnitt um die Hauptachse (A) entspricht.

10. Lenkgetriebe (10) nach zumindest einem der Ansprüche 1 bis 9,
**dadurch gekennzeichnet, dass**
der Antrieb (16), mit dem der Linearschieber (9) verbunden ist, ein Linearmotor, ein Rotationsmotor (16), der über eine Spindel (14) mit dem Linearschieber (9) verbunden ist, oder ein Hydraulik- oder Pneumatikzylinder ist.

11. Chirurgisches Instrument (1), das einen Instrumentenschaft (2), ein Werkzeug (3) an einem distalen Schaftende (2a) und eine Handhabe (5) an einem proximalen Schaftende (2b) aufweist, wobei die Handhabe (5) ein Lenkgetriebe (10) mit zumindest zwei Antrieben (16) zur Ausrichtung einer Taumelscheibe (8) aufweist, die rotativ mit einer um eine Hauptachse (A) rotierbare Hauptwelle (19) gekoppelt ist, und wobei die Taumelscheibe (8) kardanisch um ein Zentrum (Z), das auf der Hauptachse (A) liegt, gelagert und mit einer Mehrzahl Lenkdrähte (6) verbunden ist, die sich entlang der Hauptachse (A) durch den Instrumentenschaft (2) zu einer Abwinkelungsmechanik (4) des Werkzeugs (3) erstrecken,
**dadurch gekennzeichnet, dass**
das Lenkgetriebe (10) ein Lenkgetriebe (10) nach zumindest einem der Ansprüche 1 bis 10 ist.

## Claims

1. Steering gear (10) for a surgical instrument (1), the steering gear (10) comprising at least two drives (16) for spatially aligning a swash plate (8), the swash plate (8) being rotationally coupled to a main shaft (19), which is rotatable about a main axis (A), and being cardanically mounted about a center (Z) which lies on the main axis (A),
**characterized in that**
the steering gear (10) comprises at least two linear slides (9), each of which is arranged so as to be displaceable along a guide axis (Bₓ) parallel to the main axis (A) and is connected to the at least two drives (16), each linear slide (9) being operatively connected to the swash plate (8) via an engagement element pair which, as engagement elements, has an engagement opening (11, 11a, 11b, 11c) and a lever element (12) which, at a free end of a rod portion (12c), has a head portion (12a, 12b) for movable accommodation in the engagement opening (11, 11a, 11b), a pivoting plane of the swash plate (8) being defined by the head portions (12a, 12b) of the lever elements (12) and the center (Z) of the swash plate (8), and one of the engagement elements of the engagement element pair being arranged on a circumference of the swash plate (8) and the other engagement element of the engagement element pair being provided on a surface portion (9a) of the relevant linear slide (9) which faces the swash plate (8) and is a surface portion (9a) on a longitudinal side of the linear slide (9) parallel to the guide axis (Bₓ).

2. Steering gear (10) according to claim 1,
**characterized in that**
the head portion is a spherical head portion (12a) which has a spherical shape at least in part, or a T-head portion (12b) which is cylindrical at least in part.

3. Steering gear (10) according to claim 1 or 2,
**characterized in that**
the swash plate (8) comprises the engagement opening (11, 11a, 11b, 11c) and each linear slide (9) comprises one of the lever elements (12),
the engagement opening (11, 11a, 11b, 11c) being a circumferential groove (11) that extends circumferentially along the circumferential edge of the swash plate (8) or a circumferential shoulder (11c) that extends circumferentially along the circumferential edge of the swash plate (8),
and the lever element (12) being arranged on the surface portion (9a) of the relevant linear slide (9) that faces the swash plate (8), so that the rod portion (12c) points in the radial direction to the main axis (A),
and the head portion (12a, 12b) of each lever element (12) being movably accommodated in the circumferential groove (11) or in the circumferential shoulder (11c).

4. Steering gear (10) according to claim 3,
**characterized in that**
the engagement element pair further comprises, for each lever element (12) with the ball head portion (12a), a sliding block element (13) which is designed to be accommodated in the circumferentially extending circumferential groove (11) or in the circumferentially extending circumferential shoulder (11c) and has a ball socket (13a) which is designed to movably accommodate the ball head portion (12a).

5. Steering gear (10) according to claim 3 or 4,
**characterized in that**
the swash plate (8) with the circumferentially extending circumferential groove (11) is designed as an engagement opening (11, 11a, 11b, 11c) and the steering gear (10) comprises two linear slides (9), the arrangement of the lever elements (12) on the two linear slides (9) and the arrangement of the two linear slides (9) in relation to the swash plate (8) being coordinated with one another in such a way that two differently oriented pivot axes (C₁, C₂) are defined by the two head portions (12a, 12b) of the two lever elements (12) with the center (Z) of the swash plate (8), with the two pivot axes (C₁, C₂) preferably extending orthogonally to one another.

6. Steering gear (10) according to claim 3 or 4,
**characterized in that**
the swash plate (8) with the circumferentially extending circumferential shoulder (11c) is designed as an engagement opening (11, 11a, 11b, 11c) and the steering gear (10) comprises three linear slides (9) which are connected to three drives (16), the arrangement of the lever elements (12) on the three linear slides (9) and the arrangement of the three linear slides (9) in relation to the swash plate (8) being coordinated with one another in such a way that three differently oriented pivot axes (C₁, C₂, C₃) are defined by the three head portions (12a, 12b) of the three lever elements (12) with the center (Z) of the swash plate (8), with the three pivot axes (C₁, C₂, C₃) preferably each extending at an angle of 120° ± 10° to one another.

7. Steering gear (10) according to claim 3 or 4,
**characterized in that**
the swash plate (8) with the circumferentially extending circumferential shoulder (11c) is designed as an engagement opening (11, 11a, 11b, 11c) and the steering gear (10) comprises four linear slides (9),
the arrangement of the lever elements (12) on the four linear slides (9) and the arrangement of the four linear slides (9) in relation to the swash plate (8) being coordinated with one another in such a way that two pivot axes (C₁, C₂), which are each defined by two of the head portions (12a, 12b), of which the lever elements (12) are arranged opposite one another, with the center (Z) of the swash plate (8) extend orthogonally to one another,
the linear slides (9) of opposite lever elements (12) being connected in pairs to the two drives (16).

8. Steering gear (10) according to claim 2,
**characterized in that**
- at least two of the lever elements (12) are arranged on the swash plate (8), which extend from the circumferential edge of the swash plate (8) in the radial direction and are formed with the spherical head portion (12a), and
- of the at least two linear slides (9), a first linear slide (9) has a circular arc groove (11a) extending about the main axis (A) as an engagement opening (11, 11a, 11b, 11c) and a second linear slide has a cylinder bore (11b) as an engagement opening (11, 11a, 11b, 11c), which are formed in the surface portion (9a) of the relevant linear slide (9) that faces the swash plate (8),
the ball head portion (12a) of a first of the lever elements (12) being movably accommodated in the circular arc groove (11a) of the first linear slide (9) and the ball head portion (12a) of a second of the lever elements (12) being movably accommodated in the cylinder bore (11b) of the second linear slide (9),
and the arrangement of the engagement openings (11a, 11b) on the linear slides (9) and the arrangement of the at least two linear slides (9) in relation to the swash plate (8) being coordinated with the arrangement of the at least two lever elements (12) on the swash plate (8),
and the swash plate (8) having a central portion which is rotationally coupled to the main shaft (19) and rotatably mounted in a steering ring of the swash plate (8), which steering ring has the circumferential edge on which the at least two lever elements (12) are arranged.

9. Steering gear (10) according to any of claims 1 to 8,
**characterized in that**
the steering gear (10) comprises at least one housing component (15), which provides a linear guide for each linear slide (9), and/or
the surface portion (9a) is a concave surface portion (9a), which preferably corresponds to a cylinder surface portion about the main axis (A).

10. Steering gear (10) according to any of claims 1 to 9,
**characterized in that**
the drive (16) to which the linear slide (9) is connected is a linear motor, a rotary motor (16) connected to the linear slide (9) via a spindle (14), or a hydraulic or pneumatic cylinder.

11. Surgical instrument (1) comprising an instrument shaft (2), a tool (3) at a distal shaft end (2a), and a handle (5) at a proximal shaft end (2b), the handle (5) having a steering gear (10) with at least two drives (16) for aligning a swash plate (8) which is rotationally coupled to a main shaft (19), which is rotatable about a main axis (A), and the swash plate (8) being cardanically mounted about a center (Z) which lies on the main axis (A) and being connected to a plurality of steering wires (6) which extend along the main axis (A) through the instrument shaft (2) to a bending mechanism (4) of the tool (3),
**characterized in that**
the steering gear (10) is a steering gear (10) according to at least one of claims 1 to 10.

## Revendications

1. Mécanisme de direction (10) pour un instrument chirurgical (1), dans lequel le mécanisme de direction (10) présente au moins deux entraînements (16) pour l'orientation spatiale d'un plateau oscillant (8), dans lequel le plateau oscillant (8) est accouplé en rotation à un arbre principal (19) pouvant tourner autour d'un axe principal (A) et est monté par cardan autour d'un centre (Z) qui se trouve sur l'axe principal (A),
**caractérisé en ce que**
le mécanisme de direction (10) présente au moins deux coulisseaux linéaires (9) qui sont respectivement disposés de manière à pouvoir coulisser le long d'un axe de guidage (Bₓ) parallèle à l'axe principal (A) et qui sont reliés aux au moins deux entraînements (16), dans lequel chaque coulisseau linéaire (9) est en liaison active avec le plateau oscillant (8) par l'intermédiaire d'une paire d'éléments de mise en prise qui présente comme éléments de mise en prise une ouverture de mise en prise (11, 11a, 11b, 11c) et un élément de levier (12) qui présente à une extrémité libre d'une section de tige (12c) une section de tête (12a, 12b) pour la réception mobile dans l'ouverture de mise en prise (11, 11a, 11b), dans lequel un plan de pivotement du plateau oscillant (8) est défini par les sections de tête (12a, 12b) des éléments de levier (12) et le centre (Z) du plateau oscillant (8), et dans lequel l'un des éléments de mise en prise de la paire d'éléments de mise en prise est présent sur une périphérie du plateau oscillant (8) et l'autre élément de mise en prise de la paire d'éléments de mise en prise est présent sur une section de surface (9a), tournée vers le plateau oscillant (8), du coulisseau linéaire (9) respectif qui est une section de surface (9a) sur un côté longitudinal du coulisseau linéaire (9) parallèlement à l'axe de guidage (Bₓ).

2. Mécanisme de direction (10) selon la revendication 1,
**caractérisé en ce que**
la section de tête est une section de tête sphérique (12a) qui présente au moins partiellement une forme sphérique ou une section de tête en T (12b) qui est formée au moins partiellement à la manière d'un cylindre.

3. Mécanisme de direction (10) selon la revendication 1 ou 2,
**caractérisé en ce que**
le plateau oscillant (8) présente l'ouverture de mise en prise (11, 11a, 11b, 11c) et chaque coulisseau linéaire (9) présente respectivement l'un des éléments de levier (12),
dans lequel l'ouverture de mise en prise (11, 11a, 11b, 11c) est une rainure périphérique (11) s'étendant le long du bord périphérique du plateau oscillant (8) ou un épaulement périphérique (11c) s'étendant le long du bord périphérique du plateau oscillant (8),
et dans lequel l'élément de levier (12) est disposé sur la section de surface (9a), tournée vers le plateau oscillant (8), du coulisseau linéaire (9) respectif, de sorte que la section de tige (12c) est orientée dans la direction radiale par rapport à l'axe principal (A),
et dans lequel la section de tête (12a, 12b) de chaque élément de levier (12) est reçue de manière mobile dans la rainure périphérique (11) ou dans l'épaulement périphérique (11c).

4. Mécanisme de direction (10) selon la revendication 3,
**caractérisé en ce que**
la paire d'éléments de mise en prise présente en outre, pour chaque élément de levier (12) comportant la section de tête sphérique (12a), un élément formant coulisse (13) conçu pour être reçu dans la rainure périphérique (11) qui s'étend ou dans l'épaulement périphérique (11c) qui s'étend et présentant un coussinet sphérique (13a) conçu pour recevoir de manière mobile la section de tête sphérique (12a).

5. Mécanisme de direction (10) selon la revendication 3 ou 4,
**caractérisé en ce que**
le plateau oscillant (8) est conçu avec la rainure périphérique (11) qui s'étend en tant qu'ouverture de mise en prise (11, 11a, 11b, 11c) et le mécanisme de direction (10) présente deux coulisseaux linéaires (9), dans lequel la disposition des éléments de levier (12) sur les deux coulisseaux linéaires (9) et la disposition des deux coulisseaux linéaires (9) par rapport au plateau oscillant (8) sont adaptées l'une à l'autre de sorte que deux axes de pivotement (C₁, C₂) orientés différemment sont définis par les deux sections de tête (12a, 12b) des deux éléments de levier (12) avec le centre (Z) du plateau oscillant (8), dans lequel les deux axes de pivotement (C₁, C₂) s'étendent de préférence orthogonalement entre eux.

6. Mécanisme de direction (10) selon la revendication 3 ou 4,
**caractérisé en ce que**
le plateau oscillant (8) est conçu avec l'épaulement périphérique (11c) qui s'étend en tant qu'ouverture de mise en prise (11, 11a, 11b, 11c) et le mécanisme de direction (10) présente trois coulisseaux linéaires (9) qui sont reliés à trois entraînements (16), dans lequel la disposition des éléments de levier (12) sur les trois coulisseaux linéaires (9) et la disposition des trois coulisseaux linéaires (9) par rapport au plateau oscillant (8) sont adaptées l'une à l'autre de sorte que trois axes de pivotement (C₁, C₂, C₃) orientés différemment sont définis par les trois sections de tête (12a, 12b) des trois éléments de levier (12) avec le centre (Z) du plateau oscillant (8), dans lequel les trois axes de pivotement (C₁, C₂, C₃) présentent de préférence respectivement un angle de 120° ± 10° entre eux.

7. Mécanisme de direction (10) selon la revendication 3 ou 4,
**caractérisé en ce que**
le plateau oscillant (8) est conçu avec l'épaulement périphérique (11c) qui s'étend en tant qu'ouverture de mise en prise (11, 11a, 11b, 11c) et le mécanisme de direction (10) présente quatre coulisseaux linéaires (9),
dans lequel la disposition des éléments de levier (12) sur les quatre coulisseaux linéaires (9) et la disposition des quatre coulisseaux linéaires (9) par rapport au plateau oscillant (8) sont adaptés l'une à l'autre de sorte que deux axes de pivotement (C₁, C₂) définis, avec le centre (Z) du plateau oscillant (8), par respectivement deux des sections de tête (12a, 12b) dont les éléments de levier (12) sont disposés de manière opposée, s'étendent orthogonalement entre eux,
dans lequel les coulisseaux linéaires (9) d'éléments de levier (12) opposés sont reliés par paires aux deux entraînements (16).

8. Mécanisme de direction (10) selon la revendication 2,
**caractérisé en ce que**
- sur le plateau oscillant (8) sont disposés au moins deux des éléments de levier (12) qui s'étendent depuis le bord périphérique du plateau oscillant (8) dans la direction radiale et sont conçus avec la section de tête sphérique (12a), et
- parmi les au moins deux coulisseaux linéaires (9), un premier coulisseau linéaire (9) présente une rainure en arc de cercle (11a) s'étendant autour de l'axe principal (A) en tant qu'ouverture de mise en prise (11, 11a, 11b, 11c) et un deuxième coulisseau linéaire présente un alésage cylindrique (11b) en tant qu'ouverture de mise en prise (11, 11a, 11b, 11c), lesquels sont conçus dans la section de surface (9a), tournée vers le plateau oscillant (8), du coulisseau linéaire (9) respectif,
dans lequel la section de tête sphérique (12a) d'un premier des éléments de levier (12) est reçue de manière mobile dans la rainure en arc de cercle (11a) du premier coulisseau linéaire (9) et la section de tête sphérique (12a) d'un second des éléments de levier (12) est reçue de manière mobile dans l'alésage cylindrique (11b) du deuxième coulisseau linéaire (9),
et dans lequel la disposition des ouvertures de mise en prise (11a, 11b) sur les coulisseaux linéaires (9) et la disposition des au moins deux coulisseaux linéaires (9) par rapport au plateau oscillant (8) sont adaptées à la disposition des au moins deux éléments de levier (12) sur le plateau oscillant (8),
et dans lequel le plateau oscillant (8) présente une section centrale qui est accouplée en rotation à l'arbre principal (19) et qui est montée de manière à pouvoir tourner dans un anneau de direction du plateau oscillant (8) qui présente le bord périphérique sur lequel sont disposés les au moins deux éléments de levier (12).

9. Mécanisme de direction (10) selon au moins l'une des revendications 1 à 8,
**caractérisé en ce que**
le mécanisme de direction (10) présente au moins un composant de logement (15) qui fournit un guidage linéaire pour chaque coulisseau linéaire (9), et/ou
la section de surface (9a) est une section de surface concave (9a) qui correspond de préférence à une section de surface d'enveloppe cylindrique autour de l'axe principal (A).

10. Mécanisme de direction (10) selon au moins l'une des revendications 1 à 9,
**caractérisé en ce que**
l'entraînement (16) auquel le coulisseau linéaire (9) est relié est un moteur linéaire, un moteur rotatif (16) qui est relié au coulisseau linéaire (9) par l'intermédiaire d'une broche (14), ou un vérin hydraulique ou pneumatique.

11. Instrument chirurgical (1) qui présente une tige d'instrument (2), un outil (3) à une extrémité distale de tige (2a) et une poignée (5) à une extrémité proximale de tige (2b), dans lequel la poignée (5) présente un mécanisme de direction (10) comportant au moins deux entraînements (16) pour orienter un plateau oscillant (8) accouplé en rotation à un arbre principal (19) pouvant tourner autour d'un axe principal (A), et dans lequel le plateau oscillant (8) est monté par cardan autour d'un centre (Z) se trouvant sur l'axe principal (A) et est relié à une pluralité de fils de direction (6) qui s'étendent le long de l'axe principal (A) à travers la tige d'instrument (2) jusqu'à un mécanisme de coudage (4) de l'outil (3),
**caractérisé en ce que**
le mécanisme de direction (10) est un mécanisme de direction (10) selon au moins l'une des revendica tions 1 à 10.
